Europäisches Patentamt

European Patent Office

Office européen des brevets

(11) Publication number: **0 257 310 B1**

# EUROPEAN PATENT SPECIFICATION

(45) Date of publication of patent specification: **08.01.92**

(51) Int. Cl.⁵: **A61K 9/20**, A61K 9/52, A61K 9/46

(21) Application number: **87110714.0**

(22) Date of filing: **24.07.87**

(54) **Tablets made from sustained release microcapsules.**

(30) Priority: **28.07.86 US 889775**

(43) Date of publication of application:
**02.03.88 Bulletin 88/09**

(45) Publication of the grant of the patent:
**08.01.92 Bulletin 92/02**

(84) Designated Contracting States:
**CH DE FR GB IT LI**

(56) References cited:
**EP-A- 0 153 104**
**WO-A-84/02843**
**WO-A-86/04817**
**US-A- 3 488 418**
**US-A- 3 887 700**

(73) Proprietor: **Bio -Dar Ltd.**
**Kiryat Weizmann Science Park P.O. Box 2142**
**Reohvot 76121(IL)**

(72) Inventor: **Rotman, Avner**
**6 Alkalai Street**
**Rehovot(IL)**
Inventor: **Blatt, Yoav**
**29 Hertzel Street**
**Rehovot(IL)**

(74) Representative: **Schneck, Herbert, Dipl.-Phys.,**
**Dr. et al**
**Rau & Schneck Patentanwälte Königstrasse**
**2**
**W-8500 Nürnberg 1(DE)**

## Description

## FIELD OF THE INVENTION

The present invention relates to sustained release microcapsules which can be formulated into the form of a compressed tablet. The present invention further relates to tablets of sustained release microcapsules with a flexible coat which may be in a form for chewable administration. The present invention further relates to tablets of sustained release microcapsules in a form which permits rapid disintegration and effervescence upon addition to water so that the microcapsules are orally administered as an aqueous suspension.

## BACKGROUND OF THE INVENTION

Sustained release medications are well known and are very desirable in order to permit a single dose of medicament to remain effective over a period of time up to twelve hours or more. Generally, this is accomplished by means of a gelatin capsule which contains a plurality of microcapsules of medicament, each capsule being coated with a sustained release coating which permits the medicament to be slowly released through the coating of the microcapsule. Alternatively, the microcapsules may contain a plurality of different coatings, so that some of the coatings dissolve almost immediately, some do not dissolve for a number of hours, and some of the coatings prevent release of medicament for many hours.

Gelatin capsules containing medicament have fallen into disrepute in recent years, as they are subject to tampering. Foreign substances can be placed into such gelatin capsules, with unfortunate consequences. Accordingly, dosage forms are being sought which will avoid the use of capsules. This is particularly difficult with respect to sustained release medicaments, because conventional microcapsules are known to break if subjected to the pressures required to form a tablet. If the microcapsules break, then all of the medicament is released immediately, and there is no sustained release effect.

One method by which the prior art has attempted to solve this problem is described in U.S. Patents 3,922,338 and 4,113,816. In these patents, controlled release microcapsules are formulated into a tablet by sandwiching the microcapsules between two layers of excipient in granular form which serve to cushion the microcapsules of the medial layer against the shock of compression when compressing them into tablets.

Some prior art tablets achieve sustained release by mixing the drug directly with an excipient which prevents breakup of the tablets in the gastric juices or intestinal fluids so that the tablet itself slowly breaks down and releases the active ingredient over a period of time. For example, formulating the drug with a spongy polymer, such as methyl cellulose, will form a sustained release tablet which gradually releases active ingredient from the polymer. Such galenical forms have the disadvantage, however, of providing high concentrations of active principle at the spot where the tablet rests; in some cases, this can cause an ulcer or other complications. It is preferred that the tablet break up shortly after administration so as to cause the active principle to disperse and not be too heavily concentrated at one place while still achieving sustained release. No simple and effective way to attain this object has yet been found in the prior art.

WO-A-8 402 843 discloses disintegratable sustained release tablets containing the active ingredient in form of microcapsules, the flexible coat of which consists of ethylcellulose, preferably with a viscosity of 100 mPas. The size of said microcapsules lies preferably between 100 and 300 μm. These tablets possess a structure which loosens in aqueous medium but does not disintegrate to discrete particles within 4 hours.

## SUMMARY OF THE INVENTION

Accordingly, it is an object of the present invention to obviate the problems of the prior art.

It is a further object of the present invention to provide chewable tablets composed of microcapsules of sustained release medicament.

It is still a further object of the present invention to provide a sustained release tablet with immediate disintegration into many dispersed centers of sustained drug release.

It is yet a further object of the present invention to provide tablets which permit passage of the active principle immediately into the intestine without the delay of drug release in the stomach.

It is yet a further object of the present invention to provide a sustained release tablet which has a high percentage of active material.

It is still another object of the present invention to provide a tablet of compressed sustained release microcapsules which immediately disintegrates and effervesces upon addition to water so that the sustained release microcapsules may be administered as a liquid.

According to the invention microcapsules are provided having a size range distributed between 5 and 300 μm and comprising particles of active ingredient coated with an encapsulating layer of a sustained release polymer composition having sufficient flexibility that upon compression of the microcapsules into tablets, substantially no breakage of the microcapsules occurs and that the tablets comprising that microcapsules are completely disintegrated before ingestion into the stomach.

When such coated particles of medicament are compressed into a tablet, the wide range of sizes and irregular shapes of the coated particles permit sufficient compression to form a tablet without causing the coating of the medicament particles to break.

It is by using very small particles of medicament, i.e., below about 300 μm, and coating these particles with the thin, flexible coating of the present invention, that it is possible to compress the particles to a sufficient degree (for example, about 1.5 tons pressure) to form a tablet (e.g., with a hardness of about 10-20 s.c.) without substantial breakage of the microcapsules. Conventional excipients, such as starch or cellulose derivatives, may be thoroughly mixed with the coated particles prior to forming the tablet in order to enhance disintegration of the tablet, after administration, into individual microcapsules. Excipients which cause effervescence upon contact of the tablet with water may also be added.

Out of the many polymers that can be used to coat the medicaments, only long polymer length ethyl cellulose was found to be not ruptured at all during compression, even with small amounts of plasticizer. However, other sustained release polymers, such as lower viscosity ethyl cellulose and acrylic resins can be made sufficiently flexible through the use of higher amounts of plasticizer.

BRIEF DESCRIPTION OF THE DRAWINGS

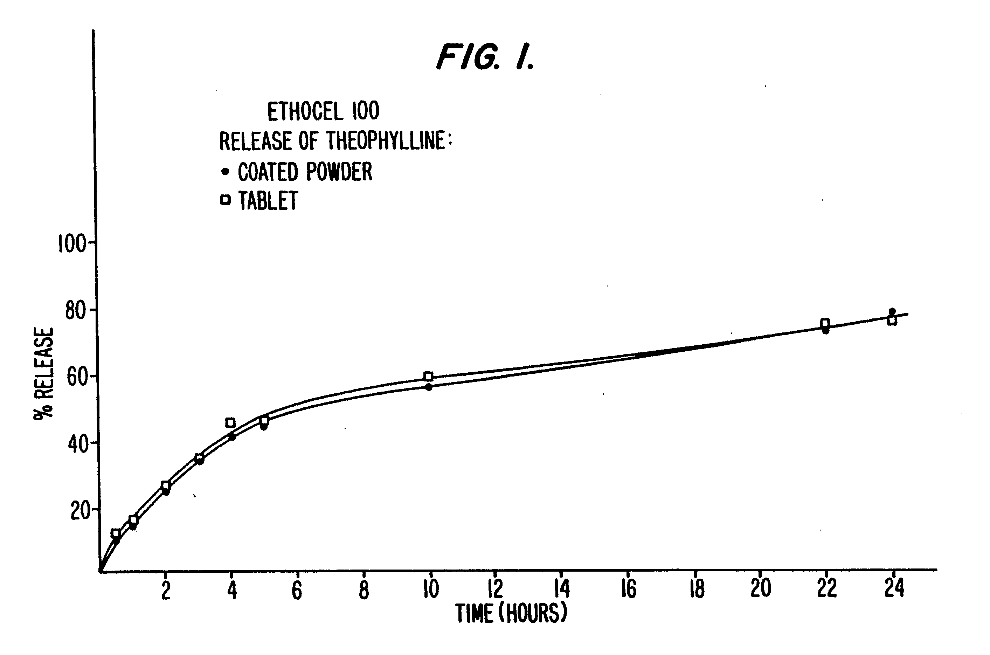

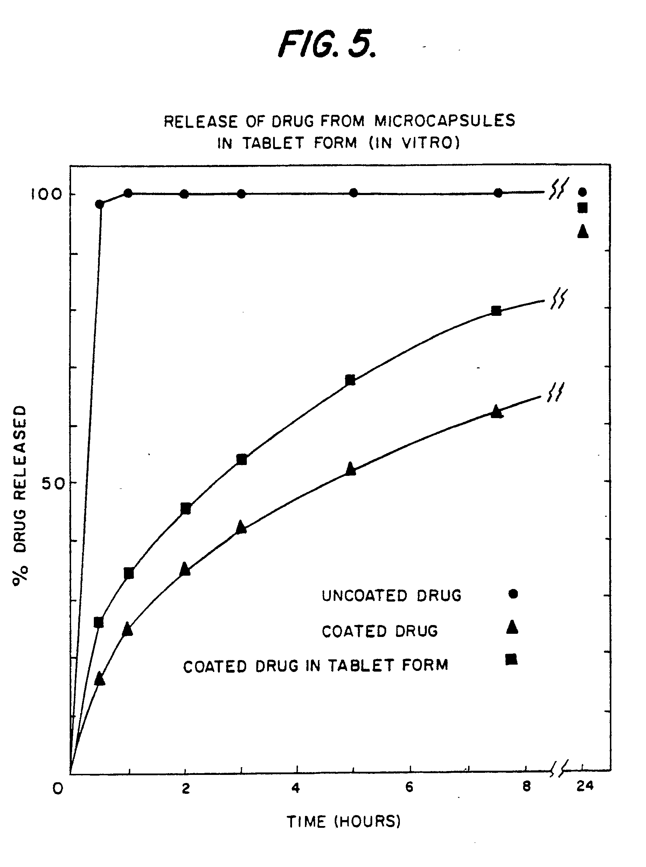

Figures 1, 2, 3, 4 and 5 show the release of a medicament from microcapsules in both powder and tablet form (in vitro) on the basis of percent drug release plotted against time.

DETAILED DESCRIPTION OF THE INVENTION

The present invention resides in a particular galenical form of administration of medicament. The particular medicament which is microencapsulated and formed into a tablet in accordance with the present invention may be totally arbitrarily selected. The utility of the present invention does not rely on the particular properties of the material being encapsulated, but rather on the particular size of the particles being encapsulated and the type of coating applied thereto. Furthermore, the active ingredient need not be a pharmaceutical, but may be any material which is desirably released over a period of time, such as a flavoring substance or an indicator substance.

Those medicaments which are most well suited to the present invention are those which are presently sold in gelatin capsules of encapsulated sustained release drug. These include appetite suppressors, such as phenylpropanolamine, antihistamines such as are present in the commercial product sold under the trademark CONTAC, sustained release aspirin, sustained release acetaminophen, ampicillin, ibuprofen, theophylline, and others. This list is merely exemplary, and the present invention is to be in no way limited by the particular active material present therein.

The particles of the active ingredient should be chosen to have a size which enables all of the coated particles to be below 300 μm in diameter, and preferably between 30 and 250 μm. Furthermore, the size should be selected to be well distributed within the entire size range. By using a wide range of different sizes of particles, which particles are of irregular shape and which shape remains irregular after coating because of the thin nature of the coating, the coated powder becomes well packed during compression, thereby minimizing breakage.

In the high viscosity ethyl cellulose embodiment of the present invention, it is important that a sufficient polymer chain length of ethyl cellulose be used for the coating in order to provide sufficient flexibility to prevent substantial breakage during compression. Ethyl cellulose is generally designated by suppliers thereof by viscosity and ethoxy content. The viscosity designation is standardized to be measured in centipoises for a 5% by weight concentration in 80:20 toluene:ethanol at 25°C on a sample which has been dried 30 minutes at 100°C. Throughout the present specification and claims, reference to viscosity of the ethyl cellulose will mean viscosity measured in accordance with the preceding standard.

It has been found that a viscosity of 100 (0,1 Pa·s) substantially eliminates all breakage of the microcapsules upon compression into tablets, as can be seen from Figure I. The use of lower viscosity ethyl cellulose decreases the flexibility of the coatings. Figure 2 shows the use of ethyl cellulose with a viscosity of 45 (0,045 Pa·s). While the results are not as good as those shown in Figure I, they are substantially better than those of Figure 3, in which the ethyl cellulose viscosity was 10 (0,01 Pa·s) and Figure 4, in which an acrylic resin sustained release coating was used. Thus, while not preferred, a viscosity as low as about 25 is still considered to be within the ambit of the present invention.

Ethyl cellulose is conventionally manufactured in various ethoxyl types. For example, medium-type products have an ethoxyl content of 45-46.8%, standard-type has an ethoxyl content of 47.5-49.5% and industrial-type has an ethoxyl content of 49.0-52.0%. While standard-type is preferred and used in the present examples, the amount of ethoxyl content does not effect the compressibility properties of the coatings of the present invention.

While ethyl cellulose has conventionally been used to form sustained release coatings for medicaments, there had never been any reason to believe that the polymer chain length of the starting ethyl cellulose would have any effect on the release properties of the final product. In fact, there is no difference in the sustained release properties of medicaments in powder form between those coated with ethyl cellulose with a viscosity of 10 and those coated with ethyl cellulose of viscosity 100. Conventionally, ethyl cellulose of a viscosity of 7 to 10 was used because of its ease of handling and solubility.

The results relating to the substantial absence of breakage after compression obtained by means of the present invention are substantially independent of the type and amount of plasticizer used with the ethyl cellulose. However, 5-15% of plasticizer is preferably used to further enhance the elasticity of the coating. An even greater amount of plasticizer, up to about 30% of the ethyl cellulose coating, can also be used if desired. Any conventional pharmaceutically acceptable plasticizer may be used, such as castor oil, dibutyl phthalate, polyethylene glycols, polypropylene glycol, citric acid esters, triacetin, etc. Other conventional plasticizers may also be used for their known film plasticizing properties.

In the increased plasticizer embodiment of the present invention, the specific nature of the coating material is not critical as long as sufficient plasticizer is present to permit the required degree of flexibility of the coating. Thus, any conventional sustained release coating, such as the acrylic resin coatings sold under the trademark Eudragit RS or RL, may be used, as can ethyl cellulose and other known sustained release coatings. The present invention is not to be limited to the particular coating used on the particles of active principle.

It is important that a relatively high percentage of plasticizer be used in the coating in order to permit sufficient flexibility to prevent substantial breakage during compression. The specific amount of plasticizer will, of course, vary depending on the nature of the coating and the particular plasticizer used. The amount may be readily determined empirically by testing the release characteristics of the tablets formed. If the medicament is being released too quickly, then more plasticizer should be used. On the other hand release characteristics are also a function of the thickness of the coating. When substantial amounts of plasticizer are used, the sustained released capacity of the coating diminishes. Thus, the thickness of the coating may be increased slightly to make up for an increase in the amount of plasticizer. It should seldom be necessary, however, to use an amount of coating which exceeds about 20 percent of the total weight of the final product. Generally, the plasticizer will be present in an amount of about 15 to 30 percent of the sustained release material in the coating, preferably 20 to 25 percent and the amount of coating will be from 10 to 25 percent of the weight of active material, preferably 15 to 20 percent.

Any conventional pharmaceutically acceptable plasticizer may be used. While castor oil is exemplified herein, the present invention is not limited thereto and other conventional plasticizers may be used for their known film plasticizing properties. Examples of other plasticizers include dibutyl phthalate, polyethylene glycols, polylpropylene glycol, citric acid esters, triacetin, etc.

In every embodiment of the present invention, the coating may be applied to the powder by techniques known per se in the prior art. The present invention does not reside in the particular technique of coating. The preferred method of coating the particles is by a fluidized bed technique using, for example, a Uni-Glatt fluidized bed powder coating apparatus. When using conventional fluidized bed techniques, any acceptable solvent, such as acetone, isopropanol, methanol, ethanol, chloroform, carbon tetrachloride, etc., may be used. To improve the flow behavior of the particles in the fluidized bed machine, glidants such as magnesium stearate, talc or Aerosil 200 can be added to the coating formula. The particular solvent and glidant and the amount used have no effect on the compressibility properties of the microcapsule, with which the present invention is concerned.

The coated particles may be compressed into tablets directly or with the addition of conventional binders, fillers, diluents, excipients, flavoring agents, lubricants, etc. Any such pharmaceutically acceptable material may be present, although it is preferred that excipients be avoided which prevent disintegration of the tablets, after administration, into their constituent coated particles. On the contrary, it may be preferred to use an excipient which causes prompt disintegration of the tablets, after administration, into many disperse centers of drug release. Known excipients which may be used for such purposes include starch and microcrystalline cellulose. Other known excipients and carriers useful for this purpose may also be used. For the embodiment in which the tablet is effervescent, small amounts of acids, such as organic acids, preferably fumaric acid and bases, such as mild bases, preferably sodium bicarbonate, may be used and flavoring substances, such as sucrose, may also be added.

The excipients and flavorings, binders, fillers, etc., are preferably thoroughly mixed with the coated particles prior to tableting. It is not necessary or desirable to sandwich the particles between layers of excipient.

When the material is ready to be compressed into tablets, it may be formed into tablets by any conventional tableting apparatus. The thin long-chain ethyl cellulose coatings on ultra small particles of a wide range of sizes, all below 300 $\mu$m, permit compression and packing into a tablet at all conventional tableting pressures without substantial breakage of the microcapsules.

A particular advantage of the use of microcapsules below 300 $\mu$m is that they may be administered in the form of a chewable tablet. Because of the very small size of the capsules, it has been discovered that very few capsules are actually broken during the act of mastication of the tablet. Thus, it is possible to administer a sustained release medicine in the form of a chewable tablet. Most conventional coated particles in commercial encapsulated microcapsules have a size range between 600 and 1000 $\mu$m. If these are chewed, there will be substantial breakage causing immediate release of what may be an overdose of medication and what is usually a very distinctive and unpleasant medicament taste. When using particles below 300 $\mu$m, the taste of the medicament may be thoroughly masked, even upon chewing, because the particles are small enough to pass into the curves and depressions of the teeth and thus avoid substantial breakage.

Another unexpected advantage of microcapsules below 300 $\mu$m each is the fact that they pass through the stomach without delay in the stomach. Because of their small size, the coated drug particles are transferred immediately into the intestines with the speed of a liquid. They need not even be enteric coated, as the vast majority will quickly pass directly into the intestine without releasing the active principle in the stomach. Larger conventional size microcapsules remain for a much longer period in the stomach, thus preventing quick release of the medicament in the intestines. Using smaller particles permits the active principle to reach the blood stream faster.

The following examples are provided for ease of illustration only, and are not intended to be limiting in any way.

## Example 1 - Ethyl Cellulose 100

Seven hundred grams of theophylline, having a crystal size ranging from 74 to 250 $\mu$m, were coated in a modified Uni-Glatt powder coater with the following coating mixture:

Ethocel 100, Dow Chemical Co.      40 g
Acetone      2200 ml
Isopropanol      550 ml
Castor oil      14 g
Magnesium stearate      14 g
Ethocel 100 is ethyl cellulose with a viscosity of 100 mPa.s (cps).

The coating conditions were as follows:
(i) Speed: 700 ml/hour
(ii) Inlet temperature: 50° C
(iii) Outlet temperature: 40-42° C
(iv) Spray pressure: 1.5 • 10⁵ N/m² (1,5 bar)

The final product contained 80 ± 0.5% theophylline.

One gram of the coated theophylline was mixed with 250 mg corn starch powder, and 250 mg of this mixture was directly compressed into tablets under 1.5 ton pressure.

The release of theophylline from this tablet was measured using simulated intestinal fluid and compared to the release of theophylline from the powder mixture before compression. The results of this are shown in Figure 1. The fact that the release characteristics are substantially identical before and after compression establishes that substantially no breakage of the microcapsules occurred during compression.

## Example 2 - Ethyl Cellulose 45

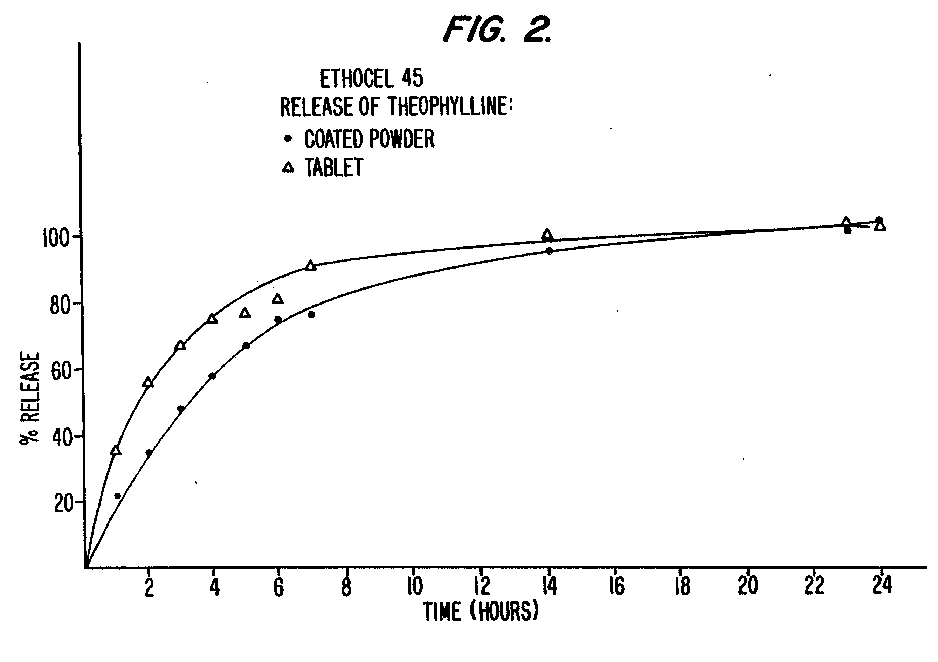

The procedure of Example 1 was followed except that the following coating mixture was used:
Ethocel 45, Dow Chemical Co.      140 g
Acetone      1750 ml
Isopropanol      40 ml
Castor oil      14 g
Magnesium stearate      14 g
Ethocel 45 is ethyl cellulose with a viscosity of 45 mPa•s (cps). Figure 2 shows the dissolution results. Lowering of the viscosity to 45 caused faster release of some of the theophylline after tableting, showing that minor breakage occurred, although, in acceptable amounts compared to conventionally used ethyl cellulose or acrylic resin coatings.

## Example 3 - Ethyl Cellulose 10 - Comparative

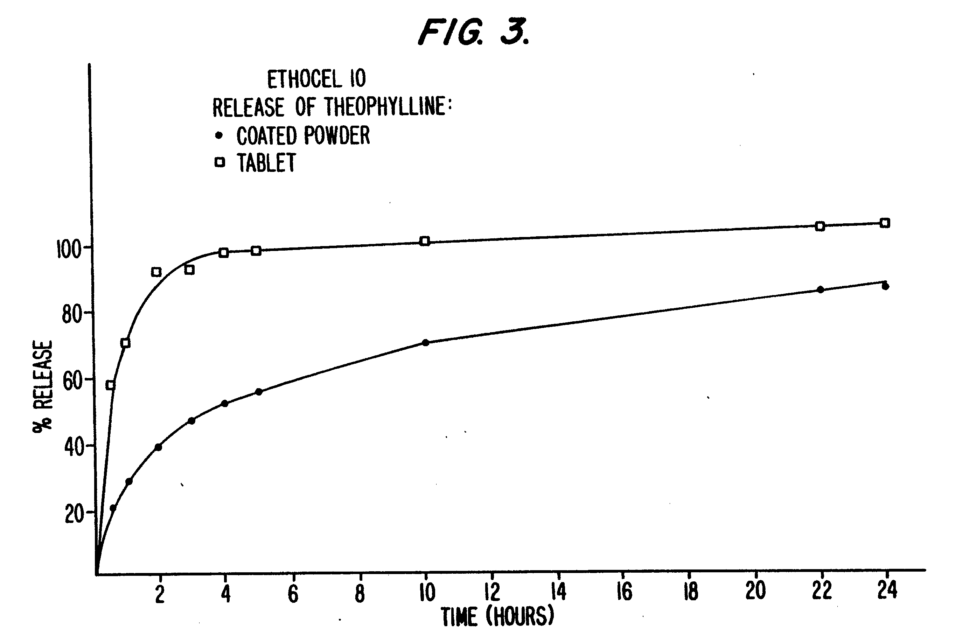

The procedure of Example 1 was followed except that the following coating mixture was used:
Ethocel 10, Dow Chemical Co.      140 g
Acetone      1400 ml
Isopropanol      300 ml
Castor oil      14 g
Magnesium stearate      14 g
Ethocel 10 is ethyl cellulose with a viscosity of 10 mPa•s cps. Fig. 3 shows the dissolution results. The curves show substantial breakage of the microcapsules upon compression.

## Example 4 - Eudragit RS-100 - Comparative

The procedure of Example I was followed except that the following coating mixture was used:

Eudragit RS-l00, Rohm Pharma, Germany     l40 g
Acetone    790 ml
Isopropanol    960 ml
Polyethylene glycol 6000    l4 g
Magnesium stearate    l4 g

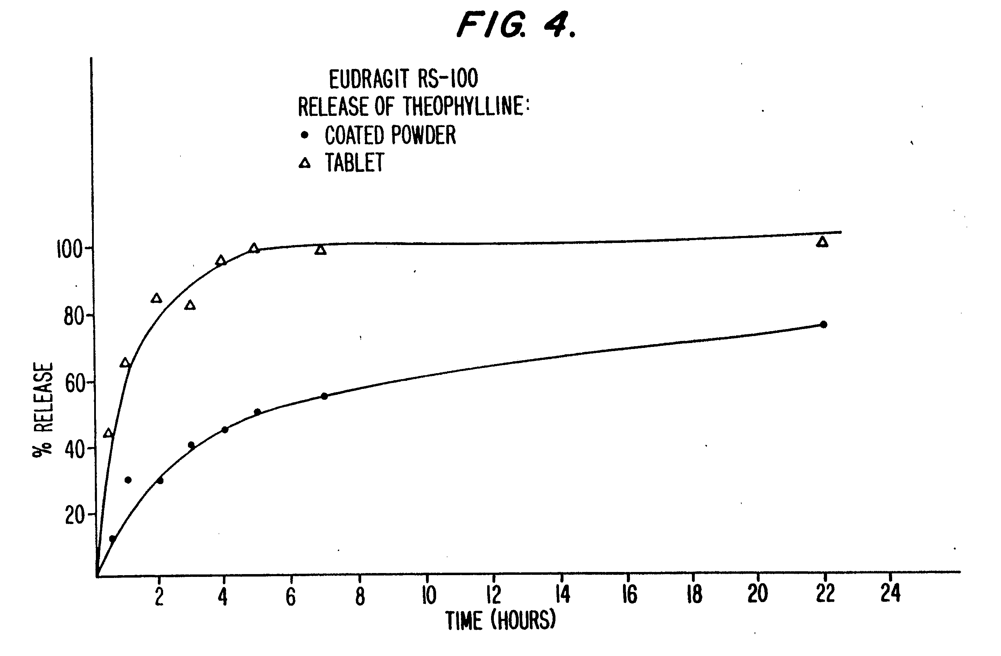

Eudragit RS-l00 is a copolymer of acrylic acid and methacrylic acid resins. The results of the dissolution are shown in Figure 4. The curves show substantial breakage of the microcapsules upon compression.

Example 5 - Effervescent Tablets

Theophylline is coated with Ethocel l00 in the same manner as described in Example I. The coated theophylline is combined into the following composition before tableting:

Coated theophylline (prepared in Example I)-    60 g
Avicel l02 (microcrystalline cellulose)    32 g
Sodium starch glycolate    2 g
Corn starch powder    l.5 g
Fumaric acid powder    l g
Sodium bicarbonate powder    l g
Sucrose powder    2 g

This mixture is directly compressed in a commercial tableting machine to a tablet of about l5 s.c. hardness. Upon being dropped into a cup of water the tablet immediately disintegrates and begins to effervesce. The effervescence causes the microcapsules to remain in suspension. Because of the microencapsulation the suspension has no taste of theophylline.

Example 6 - Ethyl Cellulose l0 and 20% Plasticizer

One kg of acetaminophen (paracetamol) was coated in a modified Uni-Glatt powder coater with ethyl cellulose. The acetaminophen had a crystal size of 74-500 $\mu$m. The ethyl cellulose used was ethyl cellulose having a viscosity of l0, obtained from Dow Chemical Company. The spraying solution comprised an 8 percent solution of the ethyl cellulose in 90 percent acetone-l0 percent ethanol. To this was added castor oil as plasticizer in an amount equal to 20 percent of the ethyl cellulose present.

The spraying conditions were as follows:
(i) Speed : l liter/hour
(ii) Flap: l0-l5 percent
(iii) Inlet Temperature: 50°C
(iv) Outlet temp.: 30°C
(v) Percent of Coating: l7 percent

The coated acetaminophen was sieved to particle sizes between 74-2l0 $\mu$m. Attention was paid to ensure a good mix of particles of different sizes within that range. 400 mg of the coated particles were mixed with l00 mg of starch and the mixture was compressed in a hand press to l.5 tons to produce 500 mg tablets.

The release characteristics of the tablets obtained in this example were tested by placing a tablet in 50 ml of simulated intestinal fluid. The tablet disintegrated within 5 to l0 seconds into many microcapsules. Similar disintegration was observed when such a tablet was added to simulated gastric fluid.

The release pattern of acetaminophen, in vitro in simulated intestinal fluid was measured. The results are shown in the drawing. The uncoated drug showed l00 percent release of the drug within a very short period of time. Coated drug, but not tableted, showed that release of the drug continued for about 24 hours with half of the drug being released in approximately l0 hours. After compression the drug was released for a total of about l6 hours with half of the drug being released after about 5 hours.

While release of the drug is slightly faster in the tableted form, a relatively small percent is immediately released, thus showing that relatively few microcapsules were broken during compression.

Experiments similar to that of this example were conducted with theophylline, phenylpropanolamine and ibuprofen and similar results were obtained.

Example 7 - Eudragit RS-l00 and 20% Plasticizer

An acrylic resin coating polymer may be prepared by dissolving l70 g Eudragit RS-l00 (Rohm Pharma, Germany) dissolved in isopropyl alcohol (60%) and acetone (40%). Eudragit RS-l00 is a sustained release acrylic resin preparation which is poorly permeable. The final polymer concentration was 8%. To this solution is added 0.5 g of pigment (Blue Lake ZLT 2), l7 g talc, and 8.5 g magnesium stearate and 34 g castor oil as plasticizer.

One kg theophylline, crystal size between 44-800$\mu$m, is coated with the acrylic resin polymer using a modified Glatt fluidized bed coating instrument (UniGlatt). The theophylline powder is fluidized and coated with the polymer. Technical details of the coating process are:

Air Flap:    25%
Inlet air temp.:    50°C
Outlet (product) temp.:    35°C
Spraying air pressure:    l.5 $\cdot 10^5$ N/m$^2$ (1,5 bar)
Pneumatic pressure:    6 $\cdot 10^5$ N/m$^2$ (6 bar)
Polymer solution feeding speed:    400ml/h
Process time:    5.5 hrs

The coated powder is sieved with a regular dual-purpose laboratory sieve shaker (Ari J. Levi Ltd.) and different fractions collected.

The obtained microcapsules in the 50-250μm range may be compressed into tablets in the same manner described with respect to Example 6.

Example 8 - Comparisons to Commercial Microcapsules

Commercial microcapsules were sampled to evaluate their size. They were sized with a regular dual-purpose laboratory sieve shaker (Ari J. Levi Ltd). The results were as follow:

1. Theotard (CTS, Israel ) 100% over 800μm
2. Dexatrim (Thompson Med., N.Y.) 95% over 800μm; 5% between 600-800μm
3. Theo-24 (Searle) 100% over 800μm
4. Eryc 250 mg (Parke-Davis) 100% over 800μm
5. Feosol (Menley & James) 100% over 800μm
6. Sudafed (Burroughs Wellcome) 100% over 800μm
7. Nitroglycerin (Ascot) 100% over 800μm
8. Thorazine (SKF) 100% over 800μm
9. Slo-phylline (Rorer) 100% over 800μm
10. Nicobid (Armour) 00% over 800μm
11. Teldrin (SKF) 30% over 800μm; 70% between 600-800μm
12. Pavabid (Marion) 100% over 800μm
13. Ornatos (Rohm Pharma, Germany)- 60% over 800μm; 40% between 600-800μm
14. Somophylline (Fisons) 97% over 800μm; 3% between 600-800μm
15. Contac (Menley & James) 33-40% over 800μm; 60-67% between 600-800μm.

Tests with tablets formed from commercial size coated particles of 600-1000 μm, such as the above, show that the pellets are completely broken by the compression and the release characteristics are substantially identical to the line on the graph for uncoated drug.

Experiment I

A test was conducted to measure the release of theophylline from various sized microcapsules using an artificial mouth. The artificial mouth used in this test was a plastic artificial mouth model manufactured by Frasaco having upper and lower jaws with teeth of natural size and configuration.

Two batches each of ten mg of coated particles were prepared as described in Example 7 but without the addition of plasticizers. One batch had a size range of 100-150μm and 600-800μm. Each contained a total of 8.3 mg theophylline. The coated particles were placed on the bottom rear teeth of the model. The model was closed and pressure was applied on the top corresponding to 2 kg for 10 sec. Then a twisting of the two rows of teeth was performed for another 15 sec. The particles (or their remains) were transferred to a test tube and the teeth were washed with 2 ml of water which was collected and transferred into the tube. The test tubes were centrifuged in a clinical centrifuge for 3 min. and the supernatant was removed. The amount of theophylline in each tube was determined by u.v. spectroscopy with the following results:

I. Large crystals (600-800μm); 2.04 mg theophylline released into the medium (24.5%)

II. Small crystals (100-150μm): 24 μg theophylline were released into the medium (0.29%)

In a control experiment it was shown that the release, due to diffusion, of theophylline through 17% coating layer of Eudragit RS-100 is less than 1% during a 5 min. period. Thus, the amount of uncoated theophylline found in the large crystals sample is due to breaking of the coated crystals by the mechanical pressure and friction of the teeth. Such a substantial release using large microcapsules is unacceptable for a sustained release dosage form. Results at least as good would be expected if the smaller microcapsules were made even more flexible by adding plasticizer.

The results of this test provide further evidence of the superior results obtainable using the preferred microcapsule size when preparing a dosage form designed to be ingested with mastication as compared to the use of microcapsules of the commercial size, particularly when the coating is a sustained release coating.

## Claims

1. A sustained release galenical form in the form of a distintegratable tablet comprising compressed microcapsules of active ingredient with a flexible coat characterized by microcapsules having a size range distributed between 5 and 300 μm and comprising particles of active ingredient coated with an encapsulating layer of an sustained release polymer composition having sufficient flexibility that upon compression of the microcapsules into tablets substantially no breakage of the microcapsules occurs and in that the tablets are completely disintegrated before ingestion into the stomach.

2. A galenical form in accordance with claim l, wherein said tablets further contain a pharmaceutically acceptable excipient interspersed among and thoroughly mixed with said microcapsules.

3. A galenical form in accordance with claim 2, wherein said excipient includes a material for causing disintegration of the tablet after administration.

4. A galenical form in accordance with claim 2, wherein said excipient includes substances which cause effervescence upon contact of said tablet with water.

5. A galenical form in accordance with claim l, wherein said microcapsules have a size range distributed between 30 and 250 μm.

6. A galenical form in accordance with claim l wherein said polymer composition comprises ethyl cellulose having a viscosity of at least 0,025 Pa·s (25 cps).

7. A galenical form in accordance with claim 5, wherein said ethyl cellulose has a viscosity of at least 0,045 Pa·s (45 cps).

8. A galenical form in accordance with claim 5, wherein said ethyl cellulose has a viscosity of at least 0,1 Pa·s (l00 cps).

9. A galenical form in accordance with claim l, wherein said polymer composition comprises a sustained release polymer containing a sufficient amount of plasticizer to render said coating flexible, said coating being no thicker than that which forms 25% of the weight of the active principle.

10. A galenical form in accordance with claim l, wherein said plasticizer is present in an amount of l5-30% of the weight of said sustained release polymer.

11. A galenical form in accordance with claim 4, wherein said plasticizer is present in an amount of about l5-20% of the weight of said sustained release polymer.

## Revendications

1. Forme galénique à libération prolongée sous la forme d'un comprimé désintégrable comprenant des microcapsules comprimées d'une substance active avec un revêtement flexible, caractérisée par des microcapsules possédant un intervalle de tailles distribuées entre 5 et 300 μm et comprenant des particules de substance active revêtues avec une couche d'encapsulage d'une composition de polymère à libération prolongée possédant une flexibilité suffisante pour que lors de la compression des microcapsules en comprimés, sensiblement aucune rupture des microcapsules ne se produise et en ce que les comprimés sont totalement désintégrables avant l'ingestion dans l'estomac.

2. Forme galénique selon la revendication 1, dans laquelle lesdits comprimés contiennent de plus un excipient pharmaceutiquement acceptable dispersé parmi lesdites microcapsules et mélangé intimement avec celles-ci.

3. Forme galénique selon la revendication 2, dans laquelle ledit excipient comprend une substance pour provoquer la désintégration du comprimé après administration.

4. Forme galénique selon la revendication 2, dans laquelle ledit excipient comprend des substances qui provoquent l'effervescence par contact dudit comprimé avec de l'eau.

5. Forme galénique selon la revendication 1, dans laquelle lesdites microcapsules possèdent un intervalle de tailles distribuées entre 30 et 250 μm.

6. Fome galénique selon la revendication 1, dans laquelle ladite composition de polymère comprend une éthylcellulose possédant une viscosité d'au moins 0,025 Pa.s (25 cps).

7. Forme galénique selon la revendication 5, dans laquelle ladite éthylcellulose possède une viscosité d'au moins 0, 045 Pa.s (45 cps).

8. Forme galénique selon la revendication 5, dans laquelle ladite éthylcellulose possède une viscosité d'au moins 0,1 Pa.s (100 cps).

9. Forme galénique selon la revendication 1, dans laquelle ladite composition de polymère comprend un polymère à libération prolongée contenant une quantité suffisante de plastifiant pour rendre ledit revêtement flexible, ledit revêtement étant d'une épaisseur telle qu'il ne forme pas plus de 25% en poids du principe actif.

10. Forme galénique selon la revendication 1, dans laquelle ledit plastifiant est présent en une quantité de 15-30% en poids dudit polymère à libération prolongée.

11. Forme galénique selon la revendication 4, dans laquelle ledit plastifiant est présent en une quantité d'environ 15-20% en poids dudit polymère à libération prolongée.

**Patentansprüche**

1. Galenische Verabreichungsform mit verzögerter Freisetzung in Form einer zersetzbaren Tablette, die komprimierte Mikrokapseln eines Wirkstoffes mit einer flexiblen Hülle aufweist, gekennzeichnet durch Mikrokapseln, die einen Größenbereich aufweisen, der zwischen 5 und 300 μm verteilt ist und die Wirkstoffpartikel aufweisen, die mit einer einkapselnden Schicht einer Polymerzusammensetzung mit verzögerter Freisetzung umhüllt sind, welche eine genügende Flexibilität hat, daß aufgrund der Pressung der Mikrokapseln in Tabletten im wesentlichen kein Bruch der Mikrokapseln auftritt, und daß die Tabletten vor Aufnahme in den Magen vollständig zerfallen sind.

2. Galenische Verabreichungsform entsprechend Anspruch 1, wobei die Tabletten weiterhin einen pharmazeutisch akzeptablen Wirkstoffträger beinhalten, der unter den Mikrokapseln verteilt und vollständig mit diesen vermischt ist.

3. Galenische Verabreichungsform entsprechend Anspruch 2, wobei der Wirkstoffträger ein Material enthält, das ein Zerfallen der Tablette nach deren Verabreichung hervorruft.

4. Galenische Verabreichungsform entsprechend Anspruch 2, wobei der Wirkstoffträger Substanzen beinhaltet, die ein Aufsprudeln aufgrund eines Kontaktes der Tablette mit Wasser hervorrufen.

5. Galenische Verabreichungsform entsprechend Anspruch 1, wobei die Mikrokapseln einen Größenbereich haben, der zwischen 30 und 250 μm aufweisen.

6. Galenische Verabreichungsform entsprechend Anspruch 1, wobei die Polymerzusammensetzung Ethylzellulose beinhaltet, die eine Viskosität von zumindest 0,025 Pa•s (25 cps) aufweist.

7. Galenische Verabreichungsform entsprechend Anspruch 5, wobei die Ethylzellulose eine Viskosität von zumindest 0,045 Pa•s (45 cps) aufweist.

8. Galenische Verabreichungsform entsprechend Anspruch 5, wobei die Ethylzellulose eine Viskosität von zumindest 0,1 Pa•s (100 cps) aufweist.

9. Galenische Verabreichungsform entsprechend Anspruch 1, wobei die Polymerzusammensetzung ein Polymer mit verzögerter Freisetzung beinhaltet, das eine genügende Menge von Weichmacher enthält, um die Umhüllung flexibel zu machen, wobei diese Umhüllung nicht dicker ist, als daß sie 25 % des Gewichtes des Wirkbestandteiles bildet.

10. Galenische Verabreichungsform entsprechend Anspruch 1, wobei der Weichmacher in einer Menge von 15 bis 30 % des Gewichtes des Polymers mit verzögerter Freisetzung vorhanden ist.

11. Galenische Verabreichungsform entsprechend Anspruch 4, wobei der Weichmacher in einer Menge von etwa 15 bis 20 % des Gewichtes des Polymers mit verzögerter Freisetzung vorhanden ist.

## FIG. I.

ETHOCEL 100
RELEASE OF THEOPHYLLINE:
- • COATED POWDER
- □ TABLET

EP 0 257 310 B1

FIG. 2.

ETHOCEL 45
RELEASE OF THEOPHYLLINE:
• COATED POWDER
△ TABLET

# FIG. 3.

ETHOCEL 10
RELEASE OF THEOPHYLLINE:
- ● COATED POWDER
- □ TABLET

% RELEASE

TIME (HOURS)

EP 0 257 310 B1

# FIG. 4.

EUDRAGIT RS-100
RELEASE OF THEOPHYLLINE:
- • COATED POWDER
- △ TABLET

EP 0 257 310 B1

# FIG. 5.

RELEASE OF DRUG FROM MICROCAPSULES
IN TABLET FORM (IN VITRO)

UNCOATED DRUG ●

COATED DRUG ▲

COATED DRUG IN TABLET FORM ■

TIME (HOURS)

% DRUG RELEASED